# EUROPÄISCHE PATENTSCHRIFT

(11) **EP 3 402 418 B1**
(45) Veröffentlichungstag und Bekanntmachung des Hinweises auf die Patenterteilung: **30.09.2020**
(21) Anmeldenummer: 17701426.3
(22) Anmeldetag: 11.01.2017
(51) Int. Cl.: A61B 17/16

(54) **MEDIZINISCHES RASPELINSTRUMENT**
MEDICAL RASP INSTRUMENT
INSTRUMENT MÉDICAL DE TYPE RUGINE

(30) Priorität: 11.01.2016 DE 102016100312
(43) Veröffentlichungstag der Anmeldung: 21.11.2018
(73) Patentinhaber: Aesculap AG, 78532 Tuttlingen (DE)
(72) Erfinder: HIRT, Martin, 78333 Stockach (DE); GÜTTLER, Thomas, 72070 Tübingen-Hirschau (DE); BADER, Christian, 78183 Hüfingen (DE)
(74) Vertreter: Hoeger, Stellrecht & Partner Patentanwälte mbB
(86) Internationale Anmeldenummer: PCT/EP2017/050450
(87) Internationale Veröffentlichungsnummer: WO 2017/121743

(56) Entgegenhaltungen:
- DE-U1-202010 000 184
- US-A- 5 041 118
- US-A1- 2010 023 014
- US-A1- 2012 265 319

## Beschreibung

Die vorliegende Erfindung betrifft ein medizinisches Instrumentarium, insbesondere zum Implantieren eines Hüftgelenkschafts, umfassend ein Raspelinstrument mit einem eine Längsachse definierenden Raspelschaft, welcher eine anteriore Seitenfläche, eine posteriore Seitenfläche, eine mediale Seitenfläche und eine laterale Seitenfläche aufweist, wobei die Seitenflächen mindestens teilweise Raspelzähne aufweisen, welche parallel zueinander verlaufend und bezogen auf die Längsachse umlaufend oder mindestens teilweise umlaufend ausgebildet sind, wobei das Raspelinstrument eine Rotationsstabilisierungseinrichtung umfasst zum Erhöhen einer Rotationsstabilität des in eine Knochenkavität, insbesondere eine Femurkavität, eingeschlagenen Raspelschafts, wobei die Rotationsstabilisierungseinrichtung eine Mehrzahl von Stabilisierungsnuten auf mindestens einer Seitenfläche des Raspelschafts umfasst und dass die Mehrzahl von Stabilisierungsnuten relativ zu den Raspelzähnen schräg verläuft, wobei die Mehrzahl von Stabilisierungsnuten auf der lateralen Seitenfläche und/ oder auf der medialen Seitenfläche und/oder auf der anterioren Seitenfläche und/oder auf der posterioren Seitenfläche des Raspelschafts ausgebildet ist.

Medizinische Instrumentarien der eingangs beschriebenen Art sind in vielfältiger Weise bekannt. Mit von solchen medizinischen Instrumentarien umfassten Raspelinstrumenten werden insbesondere Femurkavitäten präpariert, in die ein Hüftgelenkschaft einer Hüftgelenkendoprothese eingesetzt werden soll. Ein Operateur beginnt bei der Präparation der Femurkavität mit dem kleinsten verfügbaren Raspelschaft und schlägt diesen in die Femurkavität ein. Anschließend wird der Raspelschaft wieder aus der Femurkavität entnommen und ein etwas größerer Raspelschaft eingeschlagen. Diese Prozedur wird so lange wiederholt, bis die passende Größe des Raspelschafts bestimmt ist.

Zahlreiche Operateure nutzen den größten eingeschlagenen Raspelschaft, um eine Rotationsstabilität desselben im Femur zu prüfen. Dies soll eine Rotationsstabilität beispielsweise eines endgültig zu implantierenden Hüftgelenkschafts simulieren.

Ein Raspelschaft mit einer Verzahnung, die durch die eingangs beschriebenen Raspelzähne gebildet wird, die umlaufend oder mindestens teilweise umlaufend auf den Seitenflächen des Raspelschafts ausgebildet sind, zeigte sich bezüglich der Bestimmung der Rotationsstabilität bei Tests als nicht besonders gut geeignet.

Aus der DE 20 2010 000 184 U1 ist eine modulare Prothesenraspel bekannt. Eine geschlechtsspezifische Femurraspel ist in der US 2010/0023014 A1 beschrieben. Die US 5,041,118 offenbart Räumwerkzeuge für den Femur. In der US 2012/0265319 A1 ist eine Hüftgelenkendoprothese beschrieben.

Es ist daher eine Aufgabe der vorliegenden Erfindung, eine Handhabung eines medizinischen Instrumentariums der eingangs beschriebenen Art insbesondere bei der Präparation einer Knochenkavität, insbesondere eine Femurkavität, zu verbessern.

Diese Aufgabe wird bei einem medizinischen Instrumentarium der eingangs beschriebenen Art erfindungsgemäß dadurch gelöst, dass die Mehrzahl von Stabilisierungsnuten, die auf der lateralen Seitenfläche und auf der medialen Seitenfläche ausgebildet sind, alle parallel oder im Wesentlichen parallel zueinander verlaufen.

Die erfindungsgemäß vorgeschlagene Weiterbildung ermöglicht es einem Operateur insbesondere, eine Rotationsstabilität bereits mit dem eingeschlagenen Raspelschaft in der Knochenkavität, beispielsweise der Femurkavität, zu prüfen, und zwar bereits ohne den endgültig zu implantierenden Prothesenschaft einsetzen zu müssen. So hat ein Operateur den Vorteil, einerseits mit dem Raspelinstrument die Knochenkavität vorzubereiten und die passende Größe des zu implantierenden Schaftimplantats zu bestimmen und andererseits mit dem eingeschlagenen Raspelschaft passender Größe eine Rotationsstabilität des Implantatsschafts im Knochen zu überprüfen, und zwar mit dem eingeschlagenen Raspelschaft, welcher in diesem Fall quasi ein Probeimplantat bildet. Durch die Rotationsstabilisierungseinrichtung wird eine unerwünschte Rotation des Raspelschafts in der Knochenkavität minimiert, so dass auch ein Sicherheitsgefühl des Arztes bezüglich des Sitzes des Raspelschafts in der Knochenkavität erhöht wird. Bevorzugt ist eine Anzahl von Stabilisierungsnuten pro Seitenfläche, auf welcher solche Stabilisierungsnuten vorgesehen sind, größer als 6. Insbesondere können bis zu 10 oder auch noch mehr Stabilisierungsnuten, insbesondere auf der lateralen und/oder medialen Seitenfläche, ausgebildet sein. Ein Abstand der Stabilisierungsnuten voneinander ist vorzugsweise maximal so groß wie ein Abstand benachbarter Raspelzähne. Je kleiner der Abstand, umso größer eine Stabilitätswirkung der schräg verlaufenden Stabilisierungsnuten. Günstig ist es, dass die Rotationsstabilisierungseinrichtung eine Mehrzahl von Stabilisierungsnuten auf mindestens einer Seitenfläche des Raspelschafts umfasst und dass die Mehrzahl von Stabilisierungsnuten relativ zu den Raspelzähnen schräg verläuft. Eine Mehrzahl von Stabilisierungsnuten sind auf der medialen Seitenfläche und auf der lateralen Seitenfläche ausgebildet. Die schräg verlaufenden Stabilisierungsnuten können insbesondere eine Rotation des in die Knochenkavität eingeschlagenen Raspelschafts ganz oder teilweise verhindern. Auf diese Weise kann eine Rotationsstabilität des in die Knochenkavität eingeschlagenen Raspelschafts erhöht werden. Vorteilhaft ist es, dass die Mehrzahl von Stabilisierungsnuten auf der lateralen Seitenfläche und/oder auf der medialen Seitenfläche und/oder auf der anterioren Seitenfläche und/oder auf der posterioren Seitenfläche des Raspelschafts ausgebildet ist. Insbesondere ist es vorteilhaft, dass Stabilisierungsnuten auf der medialen Seitenfläche und auf der lateralen Seitenfläche ausgebildet sind. Beispielsweise können sie ausschließlich auf diesen beiden Seitenflächen ausgebildet oder vorgesehen sein. Insbesondere die mediale Seitenfläche und die laterale Seitenfläche stehen beim Präparieren der Knochenkavität mit dem Knochen in besonders großem Kontakt. Daher ist die Ausbildung der Mehrzahl von Stabilisierungsnuten auf der lateralen Seitenfläche und/oder der medialen Seitenfläche zur Verbesserung der Rotationsstabilität besonders vorteilhaft. Um insbesondere eine Rotationsstabilisierung in zueinander entgegen gesetzte Rotationsrichtungen zu erreichen, ist es günstig, dass die Mehrzahl von Stabilisierungsnuten, die auf der lateralen Seitenfläche und auf der medialen Seitenfläche ausgebildet sind, alle parallel oder im Wesentlichen parallel zueinander verlaufen. Dies bedeutet insbesondere, dass die Stabilisierungsnuten sowohl auf der lateralen Seitenfläche als auch auf der medialen Seitenfläche entweder nach anterior oder nach posterior geneigt sind, aber stets alle Stabilisierungsnuten in dieselbe Richtung. Auf diese Weise kann eine besonders hohe Rotationsstabilität erreicht werden, und zwar insbesondere unabhängig von einer Rotationsrichtung.

Eine besonders hohe Rotationsstabilität kann insbesondere dadurch erreicht werden, dass die Mehrzahl von schräg verlaufenden Stabilisierungsnuten relativ zu den Raspelzähnen unter einem Stabilisierungswinkel in einem Bereich von etwa 30° bis etwa 60° verlaufen. Günstigerweise liegt der Stabilisierungswinkel in einem Bereich von etwa 40° bis etwa 50°. Bevorzugt beträgt der Stabilisierungswinkel 45°.

Ferner kann es vorteilhaft sein, wenn die Mehrzahl von Stabilisierungsnuten, die auf der anterioren Seitenfläche und auf der posterioren Seitenfläche ausgebildet sind, alle parallel oder im Wesentlichen parallel zueinander verlaufen. Auch dies bedeutet wiederum, dass sowohl die Stabilisierungsnuten auf der anterioren Seitenfläche als auch auf der posterioren Seitenfläche entweder bezogen auf die Längsachse auf die mediale Seitenfläche hin schräg verlaufen, und zwar sowohl auf der anterioren als auch auf der posterioren Seitenfläche, oder schräg in Richtung auf die laterale Seitenfläche hin, und zwar sowohl auf der anterioren Seitenfläche als auch auf der posterioren Seitenfläche. Im Wesentlichen parallel zueinander verlaufen bedeutet hier insbesondere, dass Abweichungen von wenigen Grad zwischen einer Orientierung der parallel zueinander verlaufenden Stabilisierungsnuten auf der lateralen Seitenfläche und den parallel zueinander verlaufenden Stabilisierungsnuten auf der medialen Seitenfläche möglich sein können. Entsprechend gilt dies auch für die im Wesentlichen parallel zueinander verlaufenden Stabilisierungsnuten auf der anterioren Seitenfläche und auf der posterioren Seitenfläche.

Gemäß einer weiteren bevorzugten Ausführungsform der Erfindung kann vorgesehen sein, dass proximale Stabilisierungsnutenden der auf der lateralen Seitenfläche ausgebildeten Stabilisierungsnuten näher an der posterioren Seitenfläche positioniert sind als an der anterioren Seitenfläche und dass distale Stabilisierungsnutenden der auf der lateralen Seitenfläche ausgebildeten Stabilisierungsnuten näher an der anterioren Seitenfläche positioniert sind als an der posterioren Seitenfläche und dass die proximalen Stabilisierungsnutenden bezogen auf die Längsachse weiter proximal positioniert sind als die distalen Stabilisierungsnutenden. Eine solche Ausgestaltung ermöglicht es insbesondere, Stabilisierungsnuten auf der lateralen Seitenfläche derart auszubilden, dass sie schräg bezogen auf die Längsachse verlaufen, und zwar schräg in distaler Richtung hin von der posterioren Seitenfläche in Richtung auf die anteriore Seitenfläche hin weisend. Durch die Ausgestaltung kann eine Rotation des Raspelschafts um die Längsachse in einer Ansicht von proximal her im Gegenuhrzeigersinn wirksam stabilisiert werden.

Für eine Stabilisierung einer Rotation im Uhrzeigersinn betrachtet von proximal her ist es günstig, wenn proximale Stabilisierungsnutenden der auf der medialen Seitenfläche ausgebildeten Stabilisierungsnuten näher an der posterioren Seitenfläche positioniert sind als an der anterioren Seitenfläche und wenn distale Stabilisierungsnutenden der auf der medialen Seitenfläche ausgebildeten Stabilisierungsnuten näher an der anterioren Seitenfläche positioniert sind als an der posterioren Seitenfläche und wenn die proximalen Stabilisierungsnutenden bezogen auf die Längsachse weiter proximal positioniert sind als die distalen Stabilisierungsnutenden. Durch diese besondere Gestaltung der schräg verlaufenden Stabilisierungsnuten auf der medialen Seitenfläche kann eine Rotationsstabilisierung in einer Drehrichtung erreicht werden, die einer Rotation der oben beschriebenen Drehrichtung für die Stabilisierungsnuten auf der lateralen Seitenfläche entgegengesetzt gerichtet ist.

Gemäß einer weiteren bevorzugten Ausführungsform der Erfindung kann vorgesehen sein, dass proximale Stabilisierungsnutenden der auf der anterioren Seitenfläche ausgebildeten Stabilisierungsnuten näher an der lateralen Seitenfläche positioniert sind als an der medialen Seitenfläche und dass distale Stabilisierungsnutenden der auf der anterioren Seitenfläche ausgebildeten Stabilisierungsnuten näher an der medialen Seitenfläche positioniert sind als an der lateralen Seitenfläche und dass die proximalen Stabilisierungsnutenden bezogen auf die Längsachse weiter proximal positioniert sind als die distalen Stabilisierungsnutenden. Durch diese besondere Gestaltung der Stabilisierungsnuten auf der anterioren Seitenfläche kann eine Rotation um die Längsachse bei Draufsicht von proximal auf den Raspelschaft im Gegenuhrzeigersinn stabilisiert werden.

Für eine Rotationsstabilisierung im Uhrzeigersinn bei einer Betrachtung des Raspelschafts von proximal her ist es vorteilhaft, wenn proximale Stabilisierungsnutenden der auf der posterioren Seitenfläche ausgebildeten Stabilisierungsnuten näher an der lateralen Seitenfläche positioniert sind als an der medialen Seitenfläche und wenn distale Stabilisierungsnutenden der auf der posterioren Seitenfläche ausgebildeten Stabilisierungsnuten näher an der medialen Seitenfläche positioniert sind als an der lateralen Seitenfläche und wenn die proximalen Stabilisierungsnutenden bezogen auf die Längsachse weiter proximal positioniert sind als die distalen Stabilisierungsnutenden.

Vorzugsweise definieren die umlaufenden oder im Wesentlichen umlaufenden Raspelzähne jeweils eine Raspelzahnebene, welche quer zur Längsachse verläuft. Insbesondere kann die Raspelzahnebene senkrecht zur Längsachse des Raspelschafts verlaufen. Insbesondere dann, wenn die Längsachse des Raspelschafts gleichzeitig eine Einschlagrichtung definiert, können so die Raspelzähne besonders gut wirksam quer zur Einschlagrichtung Knochenmaterial auf Innenflächen der Knochenkavität abraspeln.

Eine besonders gute Rotationsstabilisierung lässt sich insbesondere dadurch erreichen, dass die Mehrzahl von Stabilisierungsnuten mindestens teilweise eine U-förmige oder V-förmige Querschnittsform aufweist. Ein Profil der Querschnittsform kann abhängig von der jeweiligen Seitenfläche des Raspelschafts unterschiedlich sein. Die Querschnittsform aller Stabilisierungsnuten auf allen Seitenflächen kann jedoch auch identisch sein. Dies hat insbesondere den Vorteil, dass die Stabilisierungsnuten auf einfache Weise mit einem einzigen Werkzeug, beispielsweise durch spanende Bearbeitung, ausgebildet werden können.

Vorteilhaft ist es, wenn die V-förmige Querschnittsform einen Nutöffnungswinkel zwischen relativ zueinander geneigten Nutseitenflächen einer Stabilisierungsnut definiert, welche in einem Bereich von etwa 30° bis etwa 60° liegt. Insbesondere kann der Nutöffnungswinkel in einem Bereich von etwa 40° bis etwa 50° liegen. V-förmige Stabilisierungsnuten in der definierten Weise auszubilden, ermöglicht es insbesondere, eine besonders hohe Rotationsstabilität zu erreichen. Ferner kann durch diese besondere Gestaltung der Stabilisierungsnuten insbesondere verhindert werden, dass in Folge einer Rotation des Raspelschafts um dessen Längsachse Knochenmaterial im Inneren der Knochenkavität abgeraspelt werden kann.

Insbesondere für die Herstellung des Raspelschafts ist es vorteilhaft, wenn die Mehrzahl von Stabilisierungsnuten einen verrundeten Nutgrund aufweist. So lassen sich derartige Stabilisierungsnuten beispielsweise mit einem Kegelfräser ausbilden, dessen vorderes Ende abgerundet ist.

Günstigerweise ist eine Nuttiefe der Mehrzahl von Stabilisierungsnuten auf der lateralen Seitenfläche größer als eine Zahntiefe der Raspelzähne. So kann eine besonders gute Rotationsstabilität erreicht werden.

Des Weiteren kann es vorteilhaft sein, wenn eine Nuttiefe der Mehrzahl von Stabilisierungsnuten auf der medialen Seitenfläche kleiner ist als eine Zahntiefe der Raspelzähne. Auf diese Weise können insbesondere unbeabsichtigte Abtragungen von Knochenmaterial im medialen Bereich vermeiden lassen.

Um auch das Abtragen von Knochenmaterial in der Knochenkavität nach anterior und/oder posterior zu minimieren, ist es vorteilhaft, wenn eine Nuttiefe der Mehrzahl von Stabilisierungsnuten auf der anterioren Seitenfläche und/oder auf der posterioren Seitenfläche kleiner ist als eine Zahntiefe der Raspelzähne.

Gemäß einer weiteren bevorzugten Ausführungsform der Erfindung kann vorgesehen sein, dass die mediale Seitenfläche einen proximalen Seitenflächenbereich und einen distalen Seitenflächenbereich aufweist und dass der proximale Seitenflächenbereich in einer Richtung parallel zur Längsachse vom Raspelschaft weg weisend konkav gekrümmt und in einer Richtung senkrecht zur Längsachse vom Raspelschaft weg weisend eben oder konvex gekrümmt ist. Eine solche Kontur des Raspelschafts ermöglicht eine optimale Anpassung der Knochenkavität zur Aufnahme eines entsprechend gekrümmten Prothesenschafts.

Günstig ist es, wenn die Mehrzahl von Stabilisierungsnuten der medialen Seitenfläche im proximalen oder im Wesentlichen im proximalen Seitenflächenbereich ausgebildet ist. So kann insbesondere im proximalen Seitenflächenbereich eine besonders gute Rotationsstabilität erreicht werden, also dort, wo die größten Drehmomente vom Raspelschaft auf den Knochen übertragen werden müssen.

Ferner kann es günstig sein, wenn die laterale Seitenfläche einen proximalen Seitenflächenbereich und einen distalen Seitenflächenbereich aufweist und wenn der proximale Seitenflächenbereich in einer Richtung parallel zur Längsachse vom Raspelschaft weg weisend konvex gekrümmt und in einer Richtung senkrecht zur Längsachse vom Raspelschaft weg weisend eben oder konvex gekrümmt ist. Eine solche Ausgestaltung ermöglicht eine besonders gute Anpassung der Kontur der Knochenkavität insbesondere im Bereich eines Trochanter major bei der Präparation einer Femurkavität mit dem Raspelinstrument.

Vorteilhafterweise ist die Mehrzahl von Stabilisierungsnuten der lateralen Seitenfläche im proximalen oder im Wesentlichen im proximalen Seitenflächenbereich ausgebildet. So kann insbesondere im Bereich der lateralen Seitenfläche eine besonders gute Rotationsstabilität erreicht werden, und zwar im proximalen Seitenflächenbereich der lateralen Seitenfläche.

Um eine Rotationsstabilität des Raspelschafts auch in Richtung auf ein distales Ende des Raspelschafts hin zu verbessern, ist es vorteilhaft, wenn die Mehrzahl von Stabilisierungsnuten der anterioren Seitenfläche und/oder der posterioren Seitenfläche distalseitig der proximalen Seitenflächenbereiche der lateralen Seitenfläche und/oder der medialen Seitenfläche ausgebildet sind. Insbesondere können Abstände zwischen benachbarten Stabilisierungsnuten der anterioren Seitenfläche und/oder der posterioren Seitenfläche einem mehrfachen eines Abstands benachbarter Raspelzähne entsprechen. Insbesondere kann ein Abstand dieser Stabilisierungsnuten bis zum fünffachen oder sogar noch mehr eines Abstands benachbarter Raspelzähne betragen.

Vorteilhaft kann es sein, wenn die Raspelzähne eine erste, quer oder im Wesentlichen quer zur Längsachse verlaufende und in distaler Richtung weisende Raspelzahnseitenfläche und eine zweite, bezogen auf die Längsachse geneigte und im Wesentlichen in proximaler Richtung weisende Raspelzahnseitenfläche aufweisen. Mit derartigen Raspelzähnen lässt sich eine Knochenkavität insbesondere beim Einschlagen des Raspelschafts in gewünschter Weise vergrößern.

Eine besonders gute Raspelwirkung kann insbesondere dadurch erreicht werden, dass die ersten und zweiten Raspelzahnseitenflächen zwischen sich einen Raspelzahnwinkel definieren, welcher kleiner als 90° ist. Insbesondere kann der Raspelzahnwinkel auch kleiner als 70° sein.

Für eine weitere Verbesserung einer Wirkung der Raspelzähne kann insbesondere vorgesehen sein, dass die erste Raspelzahnseitenfläche etwas in Richtung auf die Längsachse hin weisend geneigt ist zur Ausbildung einer in distaler Richtung wirkenden Hinterschneidung.

Ferner kann es günstig sein, wenn der Raspelschaft einen distalen Endabschnitt umfasst und wenn der distale Endabschnitt des Raspelschafts die Längsachse definiert. Eine solche Ausgestaltung ist insbesondere dann vorteilhaft, wenn ein proximaler Endabschnitt des Raspelschafts, welcher beispielsweise die oben definierten proximalen Seitenflächenbereiche umfasst, nicht geradlinig verläuft, sondern gekrümmt ausgebildet ist.

Eine Herstellung des Instrumentariums, insbesondere des Raspelinstruments, lässt sich beispielsweise dadurch vereinfachen, dass die anteriore Seitenfläche und/oder die posteriore Seitenfläche und/oder die laterale Seitenfläche und/oder die mediale Seitenfläche mindestens teilweise eben oder im Wesentlichen eben ausgebildet sind. Beispielsweise können die genannten Seitenflächen jeweils für sich oder in einer beliebigen Kombination ebene Flächenabschnitte aufweisen, die parallel zur Längsachse verlaufen oder aber auch schwach relativ zur Längsachse geneigt sind. Insbesondere können die distalen Seitenflächenbereiche einer oder aller vier Seitenflächen eben oder im Wesentlichen eben ausgebildet sein.

Günstig ist es, wenn am Raspelschaft im Bereich seines proximalen Endes ein erstes Kupplungselement zum lösbaren Verbinden mit einem Raspelgriff angeordnet oder ausgebildet ist. Diese Ausgestaltung ermöglicht es insbesondere, den Raspelschaft mit einem Raspelgriff zu koppeln und durch Schlagen auf den Raspelgriff den Raspelschaft in die Knochenkavität einzutreiben. Um insbesondere mehrere Raspelschäfte, beispielsweise unterschiedlicher Größe, nacheinander in die Knochenkavität einschlagen zu können, ermöglicht es das Vorsehen des ersten Kupplungselements, dass die Raspelschäfte jeweils mit demselben Raspelgriff verbunden werden können. Auf diese Weise lässt sich eine Zahl erforderlicher Teile des Instrumentariums für einen chirurgischen Eingriff verringern.

Günstig ist es, wenn das Raspelinstrument modular ausgebildet ist und einen mit dem Raspelschaft lösbar verbindbaren Raspelgriff umfasst. Wie bereits beschrieben kann der Raspelgriff ausgebildet sein, um ihn mit unterschiedlichen Raspelschäften, insbesondere unterschiedlicher Größe, verbinden zu können.

Vorzugsweise umfasst der Raspelgriff ein zweites Kupplungselement, welches in einer Kupplungsstellung mit dem ersten Kupplungselement in Eingriff steht und in einer Reinigungsstellung außer Eingriff steht. Durch das zweite Kupplungselement am Raspelgriff kann auf einfache Weise eine Einheit ausgebildet werden in Form eines Raspelinstruments, und zwar umfassend einen Raspelschaft und den Raspelgriff.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische Ansicht eines Raspelschafts in Richtung auf dessen mediale Seitenfläche und anteriore Seitenfläche;
- Figur 2:: eine perspektivische Ansicht des Raspelschafts aus Figur 1 in Richtung auf dessen laterale Seitenfläche und dessen posteriore Seitenfläche;
- Figur 3:: eine vergrößerte Ansicht des Bereichs A in Figur 1;
- Figur 4:: eine vergrößerte Ansicht des Bereichs B in Figur 2;
- Figur 5:: eine Draufsicht auf eine posteriore Seitenfläche des Raspelschafts aus Figur 1;
- Figur 6:: eine Ansicht des Raspelschafts aus Figur 5 in Richtung des Pfeils C;
- Figur 7:: eine Ansicht des Raspelschafts aus Figur 5 in Richtung des Pfeils D;
- Figur 8:: eine Draufsicht auf eine anteriore Seitenfläche des Raspelschafts aus Figur 1;
- Figur 9:: eine Ansicht des Raspelschafts aus Figur 8 in Richtung des Pfeils F;
- Figur 10:: eine Ansicht des Raspelschafts aus Figur 8 in Richtung des Pfeils E; und
- Figur 11:: eine Schnittansicht längs Linie 11-11 in Figur 9.

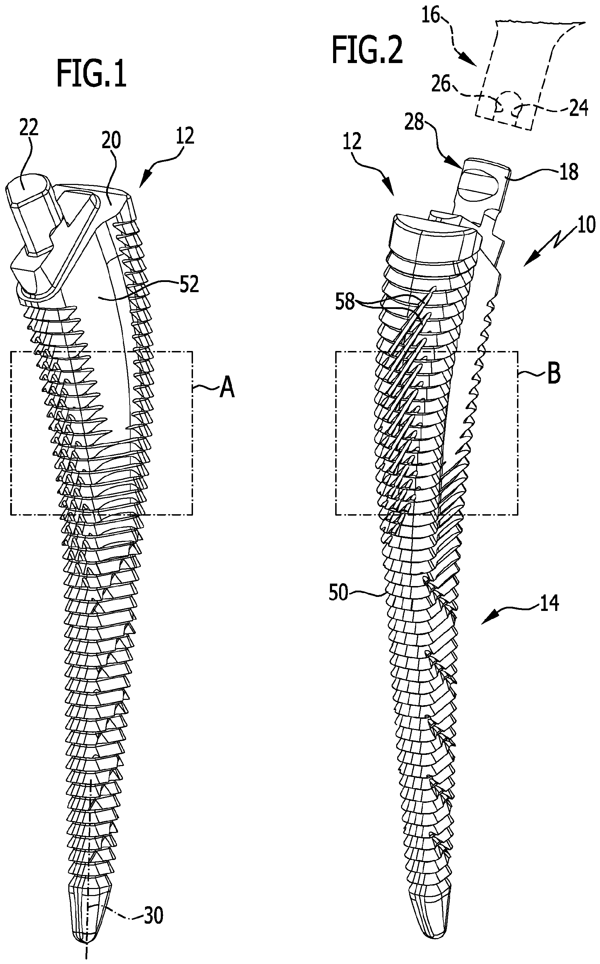

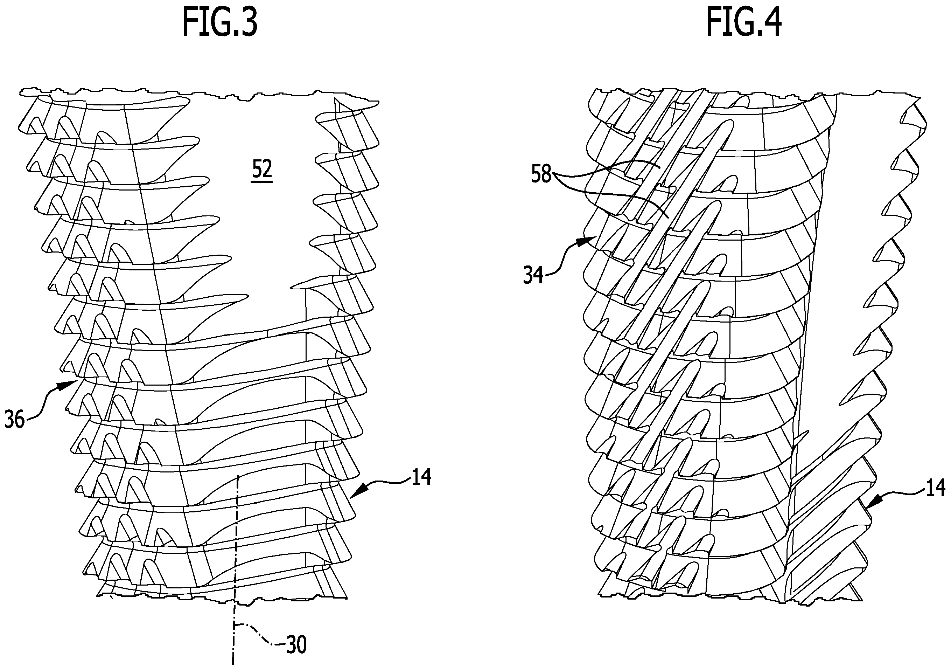

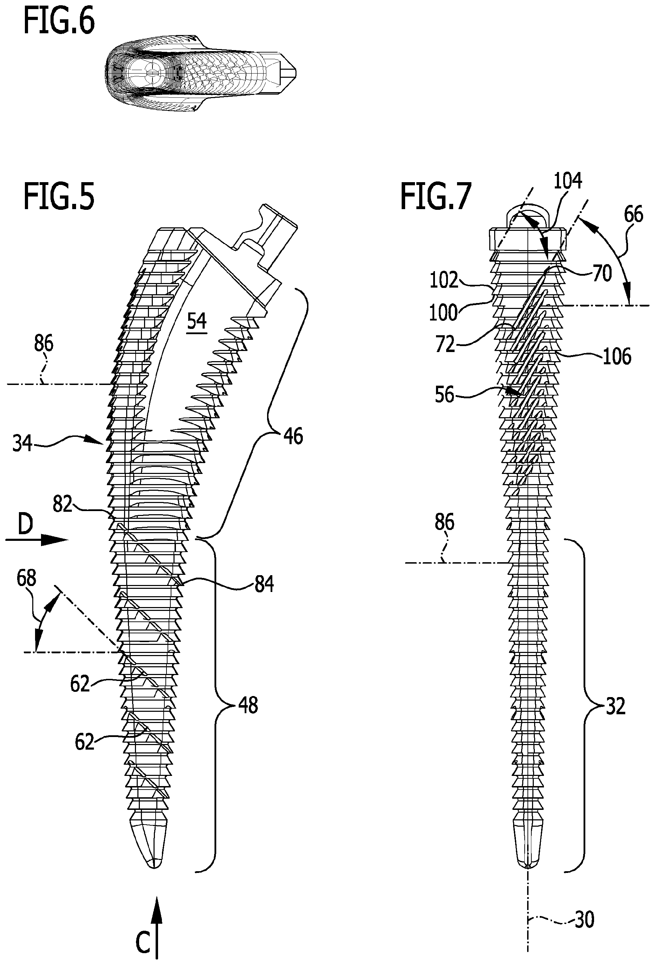

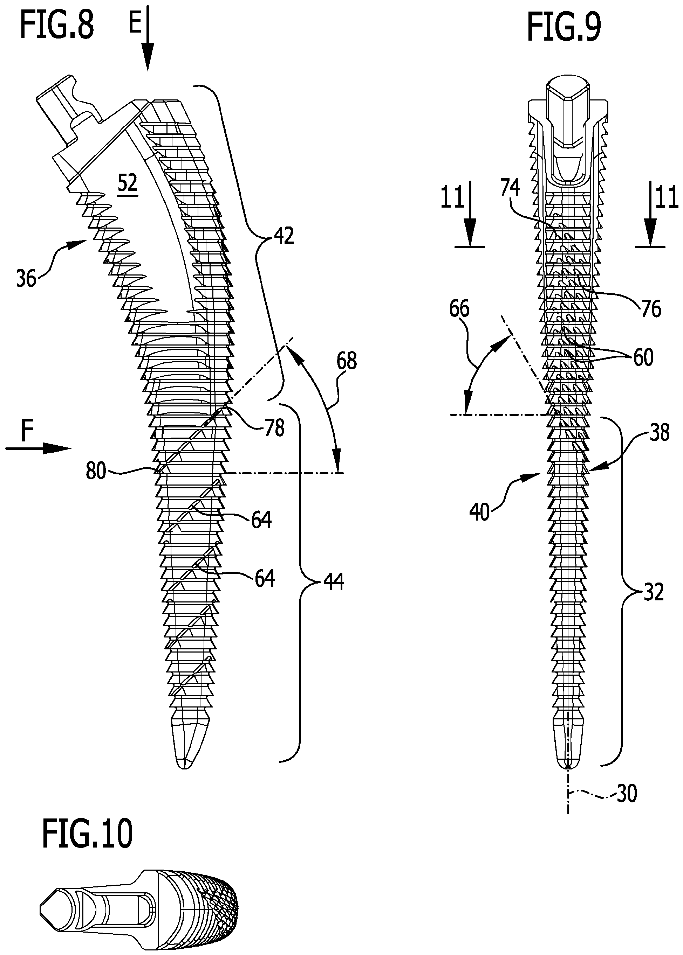

In den Figuren 1 bis 11 ist beispielhaft ein insgesamt mit dem Bezugszeichen 10 bezeichnetes Raspelinstrument eines insgesamt mit dem Bezugszeichen 12 bezeichneten medizinischen Instrumentariums dargestellt.

Das Raspelinstrument 10 umfasst einen Raspelschaft 14 und optional einen in Figur 2 schematisch dargestellten Raspelgriff 16, welcher in einer Kupplungsstellung mit dem Raspelschaft 14 verbunden ist und in einer Reinigungsstellung von diesem getrennt.

Zum lösbaren Verbinden des Raspelschafts 14 und des Raspelgriffs 16 ist von einer proximalen Endfläche 20 des Raspelschafts 14 ein erstes Kupplungselement 18 in Form eines unrunden Kupplungszapfens 22 abstehend ausgebildet.

Am Raspelgriff 16 ist ein zweites Kupplungselement 24 ausgebildet, welches mit dem ersten Kupplungselement 18 kraft- und/oder formschlüssig in Eingriff steht, so dass der Raspelschaft 14 und der Raspelgriff 16 in der Kupplungsstellung miteinander verbunden sind.

In Figur 2 ist das zweite Kupplungselement 24 schematisch als Kupplungsaufnahme 26 dargestellt.

Das erste Kupplungselement 18 und das zweite Kupplungselement 24 bilden eine insgesamt mit dem Bezugszeichen 28 bezeichnete Kupplungseinrichtung des Raspelinstruments 10.

Der Raspelschaft 10 definiert eine Längsachse 30, und zwar mit einem distalen Endabschnitt 32 desselben.

Der Raspelschaft weist insgesamt vier Seitenflächen auf, nämlich eine laterale Seitenfläche 34, eine mediale Seitenfläche 36, eine anteriore Seitenfläche 38 und eine posteriore Seitenfläche 40. Die vier genannten Seitenflächen 34, 36, 38 und 40 gehen im Wesentlichen abgerundet ineinander über. Die laterale Seitenfläche 34 weist einen proximalen Seitenflächenbereich 42 und einen distalen Seitenflächenbereich 44 auf. Der proximale Seitenflächenbereich 42 ist in einer Richtung parallel zur Längsachse 30 vom Raspelschaft 14 weg weisend konvex gekrümmt und in einer Richtung senkrecht zur Längsachse vom Raspelschaft 14 weg weisend ebenfalls konvex gekrümmt. So wird ein doppelt gekrümmter proximaler Seitenflächenbereich 42 definiert.

Die mediale Seitenfläche 36 definiert einen proximalen Seitenflächenbereich 46 und einen distalen Seitenflächenbereich 48. Der proximale Seitenflächenbereich 46 ist in einer Richtung parallel zur Längsachse 30 vom Raspelschaft 14 weg weisend konkav gekrümmt und in einer Richtung senkrecht zur Längsachse 30 vom Raspelschaft weg weisend eben oder schwach konvex gekrümmt.

Auf allen vier Seitenflächen 34, 36, 38 und 40 ist jeweils eine Mehrzahl von Raspelzähnen 50 ausgebildet, welche parallel zueinander verlaufend und bezogen auf die Längsachse 30 umlaufend oder mindestens teilweise umlaufend ausgebildet sind. Umlaufend bedeutet hier insbesondere, dass ein in sich geschlossener, sich um den Raspelschaft auf allen vier Seitenflächen 34, 36, 38, 40 erstreckender Raspelzahn ausgebildet ist.

Die Raspelzähne 50 können teilweise unterbrochen sein, beispielsweise durch einen Flächenbereich 52 der anterioren Seitenfläche 38. Dieser erstreckt sich ausgehend vom Endabschnitt 32 in distaler Richtung, jedoch nicht über die proximalen Seitenflächenbereiche 42 und 46 hinaus in distaler Richtung. Ein spiegelbildlich geformter Flächenbereich 54 frei von Stabilisierungsnuten ist auf der posterioren Seitenfläche 40 ausgebildet.

Die Besonderheit des Raspelschafts 14 bildet eine insgesamt mit dem Bezugszeichen 56 bezeichnete Rotationsstabilisierungseinrichtung zum Erhöhen einer Rotationsstabilität des in eine Knochenkavität eingeschlagenen Raspelschafts 14. Insbesondere kann es sich bei der Knochenkavität um eine Femurkavität handeln.

Die Rotationsstabilisierungseinrichtung 56 umfasst eine Mehrzahl von Stabilisierungsnuten 58, 60, 62 und 64. Die Stabilisierungsnuten 58 sind auf der lateralen Seitenfläche 34 ausgebildet, die Stabilisierungsnuten 60 auf der medialen Seitenfläche 36, die Stabilisierungsnuten 62 auf der posterioren Seitenfläche 40 und die Stabilisierungsnuten 64 auf der anterioren Seitenfläche 38.

Die Stabilisierungsnuten 58 und 60 sind jeweils in den proximalen Seitenflächenbereichen 42 beziehungsweise 46 ausgebildet.

Die Stabilisierungsnuten 62 und 64 sind jeweils distalseitig der proximalen Seitenflächenbereiche 42 und 46 ausgebildet, also in distalen Seitenflächenbereichen der posterioren und anterioren Seitenflächen 40 und 38.

Die Stabilisierungsnuten 58, 60, 62 und 64 verlaufen schräg zu den Raspelzähnen 50, und zwar unter identischen Stabilisierungswinkeln 66 zwischen den Stabilisierungsnuten 58 und den Raspelzähnen 50 beziehungsweise den Stabilisierungsnuten 60 und den Raspelzähnen 50.

Stabilisierungswinkel 68 sind zwischen den Raspelzähnen 50 und den Stabilisierungsnuten 62 einerseits und zwischen den Raspelzähnen 50 und den Stabilisierungsnuten 64 andererseits definiert.

Die Stabilisierungswinkel 66 und 68 liegen vorzugsweise in einem Bereich von etwa 30° bis etwa 60 °. Insbesondere können sie in einem Bereich von etwa 40° bis etwa 50° liegen. Idealerweise ist der Stabilisierungswinkel 66 etwas größer als der Stabilisierungswinkel 68.

Die auf der lateralen Seitenfläche 34 ausgebildeten Stabilisierungsnuten 58 weisen proximale Stabilisierungsnutenden 70 auf, die näher an der posterioren Seitenfläche 40 positioniert sind als an der anterioren Seitenfläche 38.

Distale Stabilisierungsnutenden 72 der Stabilisierungsnuten 58 sind dagegen näher an der anterioren Seitenfläche 38 positioniert als an der posterioren Seitenfläche 40. Zudem sind die proximalen Stabilisierungsnutenden 70 bezogen auf die Längsachse 30 weiter proximal positioniert als die distalen Stabilisierungsnutenden 72.

Des Weiteren sind proximale Stabilisierungsnutenden 74 der auf der medialen Seitenfläche 36 ausgebildeten Stabilisierungsnuten 60 näher an der posterioren Seitenfläche 40 positioniert als an der anterioren Seitenfläche 38.

Distale Stabilisierungsnutenden 76 der Stabilisierungsnuten 60 sind dagegen näher an der anterioren Seitenfläche 38 positioniert als an der posterioren Seitenfläche 40. Zudem sind auch die proximalen Stabilisierungsnutenden 74 bezogen auf die Längsachse 30 weiter proximal positioniert als die distalen Stabilisierungsnutenden 76.

Ferner sind proximale Stabilisierungsnutenden 78 der auf der anterioren Seitenfläche 38 ausgebildeten Stabilisierungsnuten 64 näher an der lateralen Seitenfläche 34 positioniert als an der medialen Seitenfläche 36.

Distale Stabilisierungsnutenden 80 der Stabilisierungsnuten 64 sind dagegen nähre an der medialen Seitenfläche 36 positioniert als an der lateralen Seitenfläche 34. Zudem sind die proximalen Stabilisierungsnutenden 78 bezogen auf die Längsachse 30 weiter proximal positioniert als die distalen Stabilisierungsnutenden 80.

Des Weiteren sind proximale Stabilisierungsnutenden 82 der auf der posterioren Seitenfläche 40 ausgebildeten Stabilisierungsnuten 62 näher an der lateralen Seitenfläche 34 positioniert als an der medialen Seitenfläche 36.

Ferner sind distale Stabilisierungsnutenden 84 der Stabilisierungsnuten 62 näher an der medialen Seitenfläche 36 positioniert als an der lateralen Seitenfläche 34. Zudem sind die proximalen Stabilisierungsnutenden bezogen auf die Längsachse 30 weiter proximal positioniert als die distalen Stabilisierungsnutenden 84.

Die umlaufenden oder im Wesentlichen umlaufenden Raspelzähne 50 definieren jeweils eine Raspelzahnebene 86, welche quer, bei dem in den Figuren beispielhaft dargestellten Ausführungsbeispiel des Raspelschafts 14, senkrecht zur Längsachse 30 verlaufen.

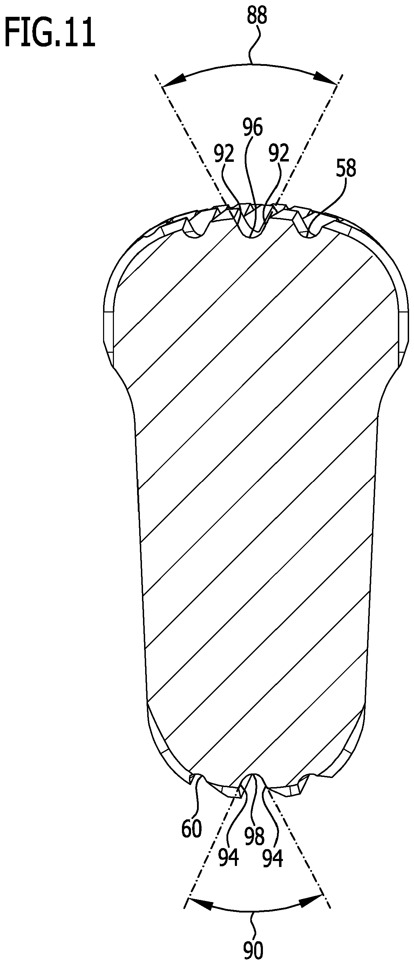

Insbesondere die Stabilisierungsnuten 58 und 60, optional auch die Stabilisierungsnuten 62 und 64, weisen mindestens teilweise eine U-förmige oder V-förmige Querschnittsform auf. Dies ist insbesondere in Figur 11 gut zu erkennen.

Die in Figur 11 schematisch dargestellte V-förmige Querschnittsform definiert einen Nutöffnungswinkel 88 zwischen relativ zueinander geneigten Nutseitenflächen 92 der Stabilisierungsnuten 58.

Ebenso definieren relativ zueinander geneigte Nutseitenflächen 94 der Stabilisierungsnuten 60 zwischen sich einen Nutöffnungswinkel 90. Die Nutöffnungswinkel 88 und 90 liegen vorzugsweise in einem Bereich von etwa 30° bis etwa 60°. Insbesondere liegen sie in einem Bereich von etwa 40° bis etwa 50°.

Die Stabilisierungsnuten 58, 60, 62 und 64 weisen jeweils einen verrundeten Nutgrund 96 beziehungsweise 98 auf.

Eine Nuttiefe der Stabilisierungsnuten 58 auf der lateralen Seitenfläche 34 ist vorzugsweise größer als eine Zahntiefe der Raspelzähne 50. Ferner ist eine Nuttiefe der Stabilisierungsnuten 60 auf der medialen Seitenfläche 36 vorzugsweise kleiner als eine Zahntiefe der Raspelzähne 50.

Des Weiteren ist eine Nuttiefe der Stabilisierungsnuten 62 und 64 auf der anterioren Seitenfläche 38 und/oder auf der posterioren Seitenfläche 40 kleiner als eine Zahntiefe der Raspelzähne 50.

Wie in den Figuren 1 und 2 gut zu erkennen, sind die Stabilisierungsnuten 60 der medialen Seitenfläche 36 im proximalen oder im Wesentlichen im proximalen Seitenflächenbereich 46 der medialen Seitenfläche 36 ausgebildet. Die Stabilisierungsnuten 58 der lateralen Seitenfläche 34 sind dagegen im proximalen oder im Wesentlichen im proximalen Seitenflächenbereich 42 der lateralen Seitenfläche 34 ausgebildet.

Die anteriore Seitenfläche 38, die posteriore Seitenfläche 40 und die laterale Seitenfläche 34 sowie die mediale Seitenfläche 36 sind mindestens teilweise eben oder im Wesentlichen eben ausgebildet. Insbesondere trifft dies auf die distalen Seitenflächenbereiche 44 und 48 aller vier Seitenflächen 34, 36, 38 und 40 zu.

Die Stabilisierungsnuten 62 und 64 der anterioren Seitenfläche 38 und der posterioren Seitenfläche 40 sind vorzugsweise distalseitig der proximalen Seitenflächenbereiche 42 und 46 der lateralen Seitenfläche 34 und der medialen Seitenfläche 36 ausgebildet.

Die Raspelzähne 50 weisen eine erste Raspelzahnseitenfläche 100 auf, die quer oder im Wesentlichen quer zur Längsachse 30 verläuft und in distaler Richtung weist. Ferner weisen die Raspelzähne 50 eine zweite Raspelzahnseitenfläche 102 auf, die bezogen auf die Längsachse geneigt ist und im Wesentlichen in proximaler Richtung weist.

Die ersten und zweiten Raspelzahnseitenflächen 100 und 102 definieren zwischen sich einen Raspelzahnwinkel 104. Dieser ist vorzugsweise kleiner als 90°, insbesondere kleiner als 70°.

Ferner kann die erste Raspelzahnseitenfläche 100 etwas in Richtung auf die Längsachse 30 hin weisend geneigt sein zur Ausbildung einer in distaler Richtung wirkenden Hinterschneidung 106.

Der Raspelschaft 14 ist vorzugsweise einstückig ausgebildet und durch spanende Bearbeitung aus einem Vollmaterial hergestellt.

Das medizinische Instrumentarium 12 kann insbesondere mehrere Raspelschäfte 14 unterschiedlicher Größe umfassen, mit denen nacheinander die Knochenkavität bearbeitet werden kann, und zwar beginnend mit dem kleinsten Raspelschaft 14. Es werden so lange sukzessive größere Raspelschäfte 14 in die Knochenkavität eingeschlagen, bis der zuletzt eingeschlagene Raspelschaft 14 in der Knochenkavität so positioniert ist, wie ein Operateur den dauerhaft zu implantierenden Implantatschaft positionieren möchte.

Der Raspelgriff 16 ist vorzugsweise mit allen Raspelschäften 14 des medizinischen Instrumentariums 12 koppelbar.

### Bezugszeichenliste

- 10: Raspelinstrument
- 12: Instrumentarium
- 14: Raspelschaft
- 16: Raspelgriff
- 18: erstes Kupplungselement
- 20: Endfläche
- 22: Kupplungszapfen
- 24: zweites Kupplungselement
- 26: Kupplungsaufnahme
- 28: Kupplungseinrichtung
- 30: Längsachse
- 32: Endabschnitt
- 34: laterale Seitenfläche
- 36: mediale Seitenfläche
- 38: anteriore Seitenfläche
- 40: posteriore Seitenfläche
- 42: proximaler Seitenflächenbereich
- 44: distaler Seitenflächenbereich
- 46: proximaler Seitenflächenbereich
- 48: distaler Seitenflächenbereich
- 50: Raspelzahn
- 52: Flächenbereich
- 54: Flächenbereich
- 56: Rotationsstabilisierungseinrichtung
- 58: Stabilisierungsnut
- 60: Stabilisierungsnut
- 62: Stabilisierungsnut
- 64: Stabilisierungsnut
- 66: Stabilisierungswinkel
- 68: Stabilisierungswinkel
- 70: proximales Stabilisierungsnutende
- 72: distales Stabilisierungsnutende
- 74: proximales Stabilisierungsnutende
- 76: distales Stabilisierungsnutende
- 78: proximales Stabilisierungsnutende
- 80: distales Stabilisierungsnutende
- 82: proximales Stabilisierungsnutende
- 84: distales Stabilisierungsnutende
- 86: Raspelzahnebene
- 88: Nutöffnungswinkel
- 90: Nutöffnungswinkel
- 92: Nutseitenfläche
- 94: Nutseitenfläche
- 96: Nutgrund
- 98: Nutgrund
- 100: erste Raspelzahnseitenfläche
- 102: zweite Raspelzahnseitenfläche
- 104: Raspelzahnwinkel
- 106: Hinterschneidung

## Patentansprüche

1. Medizinisches Instrumentarium (12), insbesondere zum Implantieren eines Hüftgelenkschafts, umfassend ein Raspelinstrument (10) mit einem eine Längsachse (30) definierenden Raspelschaft (14), welcher eine anteriore Seitenfläche (38), eine posteriore Seitenfläche (40), eine mediale Seitenfläche (36) und eine laterale Seitenfläche (34) aufweist, wobei die Seitenflächen (34, 36, 38, 40) mindestens teilweise Raspelzähne (50) aufweisen, welche parallel zueinander verlaufend und bezogen auf die Längsachse (30) umlaufend oder mindestens teilweise umlaufend ausgebildet sind, wobei das Raspelinstrument eine Rotationsstabilisierungseinrichtung (56) umfasst zum Erhöhen einer Rotationsstabilität des in eine Knochenkavität, insbesondere eine Femurkavität, eingeschlagenen Raspelschafts (14), wobei die Rotationsstabilisierungseinrichtung (56) eine Mehrzahl von Stabilisierungsnuten (58, 60, 62, 64) auf mindestens einer Seitenfläche (34, 36, 38, 40) des Raspelschafts (14) umfasst und wobei die Mehrzahl von Stabilisierungsnuten (58, 60, 62, 64) relativ zu den Raspelzähnen (50) schräg verläuft, wobei die Mehrzahl von Stabilisierungsnuten (58, 60, 62, 64) auf der lateralen Seitenfläche (34) und/ oder auf der medialen Seitenfläche (36) und/oder auf der anterioren Seitenfläche (38) und/oder auf der posterioren Seitenfläche (40) des Raspelschafts (14) ausgebildet ist, **dadurch gekennzeichnet, dass** eine Mehrzahl der Stabilisierungsnuten (58, 60) auf der lateralen Seitenfläche (34) und auf der medialen Seitenfläche (36) ausgebildet sind, wobei die Mehrzahl von Stabilisierungsnuten (58, 60), die auf der lateralen Seitenfläche (34) und auf der medialen Seitenfläche (36) ausgebildet sind, alle parallel oder im Wesentlichen parallel zueinander verlaufen.

2. Medizinisches Instrumentarium nach Anspruch 1, **dadurch gekennzeichnet, dass** die Mehrzahl von schräg verlaufenden Stabilisierungsnuten (58, 60, 62, 64) relativ zu den Raspelzähnen (50) unter einem Stabilisierungswinkel in einem Bereich von etwa 30° bis etwa 60° verlaufen, insbesondere in einem Bereich von etwa 40° bis etwa 50°.

3. Medizinisches Instrumentarium nach einem der voranstehenden Ansprüche, **dadurch gekennzeichnet, dass**
a) die Mehrzahl von Stabilisierungsnuten (62, 64), die auf der anterioren Seitenfläche (38) und auf der posterioren Seitenfläche (40) ausgebildet sind, alle parallel oder im Wesentlichen parallel zueinander verlaufen
und/oder
b) proximale Stabilisierungsnutenden der auf der lateralen Seitenfläche (34) ausgebildeten Stabilisierungsnuten (58) näher an der posterioren Seitenfläche (40) positioniert sind als an der anterioren Seitenfläche (38) und distale Stabilisierungsnutenden (72) der auf der lateralen Seitenfläche (34) ausgebildeten Stabilisierungsnuten (58) näher an der anterioren Seitenfläche (38) positioniert sind als an der posterioren Seitenfläche (40) und die proximalen Stabilisierungsnuten (70) den bezogen auf die Längsachse (30) weiter proximal positioniert sind als die distalen Stabilisierungsnutenden (72) und/oder
c) proximale Stabilisierungsnutenden (74) der auf der medialen Seitenfläche (36) ausgebildeten Stabilisierungsnuten (60) näher an der posterioren Seitenfläche (40) positioniert sind als an der anterioren Seitenfläche (38) und distale Stabilisierungsnutenden (76) der auf der medialen Seitenfläche (36) ausgebildeten Stabilisierungsnuten (60) näher an der anterioren Seitenfläche (38) positioniert sind als an der posterioren Seitenfläche (40) und die proximalen Stabilisierungsnutenden (74) bezogen auf die Längsachse (30) weiter proximal positioniert sind als die distalen Stabilisierungsnutenden (76).

4. Medizinisches Instrumentarium nach einem der voranstehenden Ansprüche, **dadurch gekennzeichnet, dass**
a) proximale Stabilisierungsnutenden (78) der auf der anterioren Seitenfläche (38) ausgebildeten Stabilisierungsnuten (64) näher an der lateralen Seitenfläche (34) positioniert sind als an der medialen Seitenfläche (36) und distale Stabilisierungsnutenden (80) der auf der anterioren Seitenfläche (38) ausgebildeten Stabilisierungsnuten (64) näher an der medialen Seitenfläche (36) positioniert sind als an der lateralen Seitenfläche (34) und die proximalen Stabilisierungsnutenden (78) bezogen auf die Längsachse (30) weiter proximal positioniert sind als die distalen Stabilisierungsnutenden (80) und/oder
b) proximale Stabilisierungsnutenden (82) der auf der posterioren Seitenfläche (40) ausgebildeten Stabilisierungsnuten (62) näher an der lateralen Seitenfläche (34) positioniert sind als an der medialen Seitenfläche (36) und distale Stabilisierungsnutenden (84) der auf der posterioren Seitenfläche (40) ausgebildeten Stabilisierungsnuten (62) näher an der medialen Seitenfläche (36) positioniert sind als an der lateralen Seitenfläche (34) und die proximalen Stabilisierungsnutenden (82) bezogen auf die Längsachse (30) weiter proximal positioniert sind als die distalen Stabilisierungsnutenden (84) und/oder
c) die umlaufenden oder im Wesentlichen umlaufenden Raspelzähne (50) jeweils eine Raspelzahnebene (86) definieren, welche quer, insbesondere senkrecht, zur Längsachse (30) verläuft.

5. Medizinisches Instrumentarium nach einem der voranstehenden Ansprüche, **dadurch gekennzeichnet, dass** die Mehrzahl von Stabilisierungsnuten (58, 60, 62, 64) mindestens teilweise eine U-förmige oder V-förmige Querschnittsform aufweist,
wobei insbesondere die V-förmige Querschnittsform einen Nutöffnungswinkel (88, 90) zwischen relativ zueinander geneigten Nutseitenflächen (92, 94) einer Stabilisierungsnut (58) definiert, welcher in einem Bereich von etwa 30° bis etwa 60° liegt, insbesondere in einem Bereich von etwa 40° bis etwa 50°.

6. Medizinisches Instrumentarium nach einem der voranstehenden Ansprüche, **dadurch gekennzeichnet, dass**
a) die Mehrzahl von Stabilisierungsnuten (58, 60, 62, 64) einen verrundeten Nutgrund (96, 98) aufweist
und/oder
b) eine Nuttiefe der Mehrzahl von Stabilisierungsnuten (58) auf der lateralen Seitenfläche (34) größer ist als eine Zahntiefe der Raspelzähne (50)
und/oder
c) eine Nuttiefe der Mehrzahl von Stabilisierungsnuten (60) auf der medialen Seitenfläche (36) kleiner ist als eine Zahntiefe der Raspelzähne (50)
und/oder
d) eine Nuttiefe der Mehrzahl von Stabilisierungsnuten (62, 64) auf der anterioren Seitenfläche (38) und/oder auf der posterioren Seitenfläche (40) kleiner ist als eine Zahntiefe der Raspelzähne (50).

7. Medizinisches Instrumentarium nach einem der voranstehenden Ansprüche, **dadurch gekennzeichnet, dass** die mediale Seitenfläche (36) einen proximalen Seitenflächenbereich (46) und einen distalen Seitenflächenbereich (48) aufweist und dass der proximale Seitenflächenbereich (46) in einer Richtung parallel zur Längsachse (30) vom Raspelschaft (14) weg weisend konkav gekrümmt und in einer Richtung senkrecht zur Längsachse (30) vom Raspelschaft (14) weg weisend eben oder konvex gekrümmt ist.

8. Medizinisches Instrumentarium nach Anspruch 7, **dadurch gekennzeichnet, dass**
a) die Mehrzahl von Stabilisierungsnuten (60) der medialen Seitenfläche (36) im proximalen oder im Wesentlichen im proximalen Seitenflächenbereich (46) ausgebildet ist
und/oder
b) die Mehrzahl von Stabilisierungsnuten (62, 64) der anterioren Seitenfläche (38) und/oder der posterioren Seitenfläche (40) distalseitig der proximalen Seitenflächenbereiche (42, 46) der lateralen Seitenfläche (34) und/oder der medialen Seitenfläche (36) ausgebildet sind.

9. Medizinisches Instrumentarium nach einem der voranstehenden Ansprüche, **dadurch gekennzeichnet, dass** die laterale Seitenfläche (34) einen proximalen Seitenflächenbereich (42) und einen distalen Seitenflächenbereich (44) aufweist und dass der proximale Seitenflächenbereich (42) in einer Richtung parallel zur Längsachse (30) vom Raspelschaft (14) weg weisend konvex gekrümmt und in einer Richtung senkrecht zur Längsachse (30) vom Raspelschaft (14) weg weisend eben oder konvex gekrümmt ist,
wobei insbesondere die Mehrzahl von Stabilisierungsnuten (58) der lateralen Seitenfläche (34) im proximalen oder im Wesentlichen im proximalen Seitenflächenbereich (42) ausgebildet ist.

10. Medizinisches Instrumentarium nach einem der voranstehenden Ansprüche, **dadurch gekennzeichnet, dass** die Raspelzähne (50) eine erste, quer oder im Wesentlichen quer zur Längsachse (30) verlaufende und in distaler Richtung weisende Raspelzahnseitenfläche (100) und eine zweite, bezogen auf die Längsachse (30) geneigte und im Wesentlichen in proximaler Richtung weisende Raspelzahnseitenfläche (102) aufweisen.

11. Medizinisches Instrumentarium nach Anspruch 10, **dadurch gekennzeichnet, dass**
a) die ersten und zweiten Raspelzahnseitenflächen (100, 102) zwischen sich einen Raspelzahnwinkel (104) definieren, welcher kleiner als 90°, insbesondere kleiner als 70°, ist
und/oder
b) die erste Raspelzahnseitenfläche (100) etwas in Richtung auf die Längsachse (30) hin weisend geneigt ist zur Ausbildung einer in distaler Richtung wirkenden Hinterschneidung (106).

12. Medizinisches Instrumentarium nach einem der voranstehenden Ansprüche, **dadurch gekennzeichnet, dass** der Raspelschaft (14) einen distalen Endabschnitt (32) umfasst und dass der distale Endabschnitt (32) des Raspelschafts (14) die Längsachse (30) definiert.

13. Medizinisches Instrumentarium nach einem der voranstehenden Ansprüche, **dadurch gekennzeichnet, dass**
a) die anteriore Seitenfläche (38) und/oder die posteriore Seitenfläche (40) und/oder die laterale Seitenfläche (34) und/oder die mediale Seitenfläche (36) mindestens teilweise eben oder im Wesentlichen eben ausgebildet sind
und/oder
b) am Raspelschaft (14) im Bereich seines proximalen Endes ein erstes Kupplungselement (18) zum lösbaren Verbinden mit einem Raspelgriff (16) angeordnet oder ausgebildet ist.

14. Medizinisches Instrumentarium nach einem der voranstehenden Ansprüche, **dadurch gekennzeichnet, dass** das Raspelinstrument (10) modular ausgebildet ist und einen mit dem Raspelschaft (14) lösbar verbindbaren Raspelgriff (16) umfasst.

15. Medizinisches Instrumentarium nach Anspruch 14, **dadurch gekennzeichnet, dass** der Raspelgriff (16) ein zweites Kupplungselement (24) umfasst, welches in einer Kupplungsstellung mit dem ersten Kupplungselement (18) in Eingriff steht und in einer Reinigungsstellung außer Eingriff steht.

## Claims

1. Medical instrumentarium (12), in particular for implanting a hip joint stem, comprising a rasp instrument (10) with a rasp shank (14) defining a longitudinal axis (30), which rasp shank (14) has an anterior side face (38), a posterior side face (40), a medial side face (36), and a lateral side face (34), wherein the side faces (34, 36, 38, 40) at least partially have rasp teeth (50) which are formed running parallel to each other and circumferential or at least partially circumferential with respect to the longitudinal axis (30), wherein the rasp instrument comprises a rotational stabilization device (56) for increasing a rotational stability of the rasp shank (14) driven into a bone cavity, in particular a femur cavity, wherein the rotational stabilization device (56) comprises a multitude of stabilization grooves (58, 60, 62, 64) on at least one side face (34, 36, 38, 40) of the rasp shank (14) and wherein the multitude of stabilization grooves (58, 60, 62, 64) runs obliquely relative to the rasp teeth (50), wherein the multitude of stabilization grooves (58, 60, 62, 64) is formed on the lateral side face (34) and/or on the medial side face (36) and/or on the anterior side face (38) and/or on the posterior side face (40) of the rasp shank (14), **characterized in that** a multitude of stabilization grooves (58, 60) is formed on the lateral side face (34) and on the medial side face (36), wherein the multitude of stabilization grooves (58, 60) that are formed on the lateral side face (34) and on the medial side face (36) all run parallel or substantially parallel to each other.

2. Medical instrumentarium according to Claim 1, **characterized in that** the multitude of stabilization grooves (58, 60, 62, 64) running obliquely relative to the rasp teeth (50) run at a stabilization angle in a range from about 30° to about 60°, in particular in a range from about 40° to about 50°.

3. Medical instrumentarium according to any one of the preceding Claims, **characterized in that**
a) the multitude of stabilization grooves (62, 64) that are formed on the anterior side face (38) and on the posterior side face (40) all run parallel or substantially parallel to each other
and/or
b) proximal stabilization groove ends of the stabilization grooves (58) formed on the lateral side face (34) are positioned closer to the posterior side face (40) than to the anterior side face (38), and distal stabilization groove ends (72) of the stabilization grooves (58) formed on the lateral side face (34) are positioned closer to the anterior side face (38) than to the posterior side face (40), and the proximal stabilization grooves (70) are positioned further proximally with respect to the longitudinal axis (30) than the distal stabilization groove ends (72)
and/or
c) proximal stabilization groove ends (74) of the stabilization grooves (60) formed on the medial side face (36) are positioned closer to the posterior side face (40) than to the anterior side face (38), and distal stabilization groove ends (76) of the stabilization grooves (60) formed on the medial side face (36) are positioned closer to the anterior side face (38) than to the posterior side face (40), and the proximal stabilization groove ends (74) are positioned further proximally with respect to the longitudinal axis (30) than the distal stabilization groove ends (76).

4. Medical instrumentarium according to any one of the preceding Claims, **characterized in that**
a) proximal stabilization groove ends (78) of the stabilization grooves (64) formed on the anterior side face (38) are positioned closer to the lateral side face (34) than to the medial side face (36), and distal stabilization groove ends (80) of the stabilization grooves (64) formed on the anterior side face (38) are positioned closer to the medial side face (36) than to the lateral side face (34), and the proximal stabilization groove ends (78) are positioned further proximally with respect to the longitudinal axis (30) than the distal stabilization groove ends (80)
and/or
b) proximal stabilization groove ends (82) of the stabilization grooves (62) formed on the posterior side face (40) are positioned closer to the lateral side face (34) than to the medial side face (36), and distal stabilization groove ends (84) of the stabilization grooves (62) formed on the posterior side face (40) are positioned closer to the medial side face (36) than to the lateral side face (34), and the proximal stabilization groove ends (82) are positioned further proximally with respect to the longitudinal axis (30) than the distal stabilization groove ends (84)
and/or
c) the circumferential or substantially circumferential rasp teeth (50) each define a rasp tooth plane (86) which runs transversely, in particular perpendicularly, to the longitudinal axis (30).

5. Medical instrumentarium according to any one of the preceding Claims, **characterized in that** the multitude of stabilization grooves (58, 60, 62, 64) at least partially has a U-shaped or V-shaped cross-sectional shape,
wherein in particular the V-shaped cross-sectional shape defines a groove opening angle (88, 90) between groove side faces (92, 94) of a stabilization groove (58) tilted relative to each other, which is in a range from about 30° to about 60°, in particular in a range from about 40° to about 50°.

6. Medical instrumentarium according to any one of the preceding Claims, **characterized in that**
a) the multitude of stabilization grooves (58, 60, 62, 64) has a chamfered groove base (96, 98)
and/or
b) a groove depth of the multitude of stabilization grooves (58) on the lateral side face (34) is greater than a tooth depth of the rasp teeth (50)
and/or
c) a groove depth of the multitude of stabilization grooves (60) on the medial side face (36) is less than a tooth depth of the rasp teeth (50)
and/or
d) a groove depth of the multitude of stabilization grooves (62, 64) on the anterior side face (38) and/or on the posterior side face (40) is less than a tooth depth of the rasp teeth (50).

7. Medical instrumentarium according to any one of the preceding Claims, **characterized in that** the medial side face (36) has a proximal side face region (46) and a distal side face region (48), and **in that** the proximal side face region (46) is concavely curved pointing away from the rasp shank (14) in a direction parallel to the longitudinal axis (30) and is planar or convexly curved pointing away from the rasp shank (14) in a direction perpendicular to the longitudinal axis (30).

8. Medical instrumentarium according to Claim 7, **characterized in that**
a) the multitude of stabilization grooves (60) of the medial side face (36) is formed in the proximal or substantially in the proximal side face region (46)
and/or
b) the multitude of stabilization grooves (62, 64) of the anterior side face (38) and/or of the posterior side face (40) are formed on the distal side of the proximal side face regions (42, 46) of the lateral side face (34) and/or of the medial side face (36).

9. Medical instrumentarium according to any one of the preceding Claims, **characterized in that** the lateral side face (34) has a proximal side face region (42) and a distal side face region (44), and **in that** the proximal side face region (42) is convexly curved pointing away from the rasp shank (14) in a direction parallel to the longitudinal axis (30) and is planar or convexly curved pointing away from the rasp shank (14) in a direction perpendicular to the longitudinal axis (30),
wherein in particular the multitude of stabilization grooves (58) of the lateral side face (34) is formed in the proximal or substantially in the proximal side face region (42).

10. Medical instrumentarium according to any one of the preceding Claims, **characterized in that** the rasp teeth (50) have a first rasp tooth side face (100) running transversely or substantially transversely to the longitudinal axis (30) and pointing in distal direction and a second rasp tooth side face (102) tilted with respect to the longitudinal axis (30) and pointing substantially in proximal direction.

11. Medical instrumentarium according to Claim 10, **characterized in that**
a) the first and second rasp tooth side faces (100, 102) define between them a rasp tooth angle (104) which is less than 90°, in particular less than 70°,
and/or
b) the first rasp tooth side face (100) is tilted pointing somewhat in the direction toward the longitudinal axis (30) for forming an undercut (106) effective in the distal direction.

12. Medical instrumentarium according to any one of the preceding Claims, **characterized in that** the rasp shank (14) comprises a distal end section (32), and **in that** the distal end section (32) of the rasp shank (14) defines the longitudinal axis (30).

13. Medical instrumentarium according to any one of the preceding Claims, **characterized in that**
a) the anterior side face (38) and/or the posterior side face (40) and/or the lateral side face (34) and/or the medial side face (36) are formed at least partially planar or substantially planar and/or
b) a first coupling element (18) is arranged or formed on the rasp shank (14) in the region of its proximal end for detachably connecting to a rasp grip (16).

14. Medical instrumentarium according to any one of the preceding Claims, **characterized in that** the rasp instrument (10) is modularly formed and comprises a rasp grip (16) that is detachably connectable to the rasp shank (14).

15. Medical instrumentarium according to Claim 14, **characterized in that** the rasp grip (16) comprises a second coupling element (24) which, in a coupling position, is engaged with the first coupling element (18) and, in a cleaning position, is disengaged.

## Revendications

1. Instrumentation médicale (12), en particulier pour implanter une tige d'articulation de la hanche, comprenant un instrument de râpe (10) avec une tige de râpe (14) définissant un axe longitudinal (30), qui présente une surface de côté antérieure (38), une surface de côté postérieure (40), une surface de côté médiane (36) et une surface de côté latérale (34), où les surfaces de côté (34, 36, 38, 40) présentent au moins en partie des dents de râpe (50) qui sont formées en s'étendant parallèlement entre elles et sont formées de manière circonférentielle ou au moins en partie circonférentielle par rapport à l'axe longitudinal (30), où l'instrument de râpe comprend un dispositif de stabilisation en rotation (56) pour augmenter une stabilité en rotation de la tige de râpe (14) introduite par battage dans une cavité osseuse, en particulier une cavité fémorale, où le dispositif de stabilisation en rotation (56) comprend une pluralité de rainures de stabilisation (58, 60, 62, 64) sur au moins une surface de côté (34, 36, 38, 40) de la tige de râpe (14) et où la pluralité de rainures de stabilisation (58, 60, 62, 64) s'étendent obliquement par rapport aux dents de râpe (50), où la pluralité de rainures de stabilisation (58, 60, 62, 64) sont formées sur la surface de côté latérale (34) et/ou sur la surface de côté médiane (36) et/ou sur la surface de côté antérieure (38) et/ou sur la surface de côté postérieure (40) de la tige de râpe (14), **caractérisée en ce qu'**une pluralité des rainures de stabilisation (58, 60) sont formées sur la surface de côté latérale (34) et sur la surface de côté médiane (36), où la pluralité de rainures de stabilisation (58, 60) qui sont formées sur la surface de côté latérale (34) et sur la surface de côté médiane (36), s'étendent toutes parallèlement ou sensiblement parallèlement entre elles.

2. Instrumentation médicale selon la revendication 1, **caractérisée en ce que** la pluralité de rainures de stabilisation (58, 60, 62, 64) qui s'étendent obliquement s'étendent par rapport aux dents de râpe (50) sous un angle de stabilisation dans une plage d'environ 30° à environ 60°, en particulier dans une plage d'environ 40° à environ 50°.

3. Instrumentation médicale selon l'une des revendications précédentes, **caractérisée en ce que**
a) la pluralité de rainures de stabilisation (62, 64) qui sont formées sur la surface de côté antérieure (38) et sur la surface de côté postérieure (40) s'étendent toutes parallèlement ou sensiblement parallèlement entre elles
et/ou
b) les extrémités de rainures de stabilisation proximales des rainures de stabilisation (58) formées sur la surface de côté latérale (34) sont positionnées plus près de la surface de côté postérieure (40) que de la surface de côté antérieure (38) et les extrémités de rainures de stabilisation distales (72) des rainures de stabilisation (58) formées sur la surface de côté latérale (34) sont positionnées plus près de la surface de côté antérieure (38) que de la surface de côté postérieure (40) et les rainures de stabilisation proximales (70) sont positionnées de manière plus proximale par rapport à l'axe longitudinal (30) que les extrémités de rainures de stabilisation distales (72)
et/ou
c) les extrémités de rainures de stabilisation proximales (74) des rainures de stabilisation (60) formées sur la surface de côté médiane (36) sont positionnées plus près de la surface de côté postérieure (40) que de la surface de côté antérieure (38) et les extrémités de rainures de stabilisation distales (76) des rainures de stabilisation (60) formées sur la surface de côté médiane (36) sont positionnées plus près de la surface de côté antérieure (38) que de la surface de côté postérieure (40) et les extrémités de rainures de stabilisation proximales (74) sont positionnées de manière plus proximale par rapport à l'axe longitudinal (30) que les extrémités de rainures de stabilisation distales (76).

4. Instrumentation médicale selon l'une des revendications précédentes, **caractérisée en ce que**
a) les extrémités de rainures de stabilisation proximales (78) des rainures de stabilisation (64) formées sur la surface de côté antérieure (38) sont positionnées plus près de la surface de côté latérale (34) que de la surface de côté médiane (36) et les extrémités de rainures de stabilisation distales (80) des rainures de stabilisation (64) formées sur la surface de côté antérieure (38) sont positionnées plus près de la surface de côté médiane (36) que de la surface de côté latérale (34) et les extrémités de rainures de stabilisation proximales (78) sont positionnées de manière plus proximale par rapport à l'axe longitudinal (30) que les extrémités de rainures de stabilisation distales (80)
et/ou
b) les extrémités de rainures de stabilisation proximales (82) des rainures de stabilisation (62) formées sur la surface de côté postérieure (40) sont positionnées plus près de la surface de côté latérale (34) que de la surface de côté médiane (36) et les extrémités de rainures de stabilisation distales (84) des rainures de stabilisation (62) formées sur la surface de côté postérieure (40) sont positionnées plus près de la surface de côté médiane (36) que de la surface de côté latérale (34) et les extrémités de rainures de stabilisation proximales (82) sont positionnées de manière plus proximale par rapport à l'axe longitudinal (30) que les extrémités de rainures de stabilisation distales (84)
et/ou
c) les dents de râpe circonférentielles ou sensiblement circonférentielles (50) définissent dans chaque cas un plan de dents de râpe (86) qui s'étend transversalement, en particulier perpendiculairement, à l'axe longitudinal (30).

5. Instrumentation médicale selon l'une des revendications précédentes, **caractérisée en ce que** la pluralité de rainures de stabilisation (58, 60, 62, 64) présente au moins en partie une forme en section transversale en forme de U ou en forme de V,
où en particulier la section transversale en forme de V définit entre les surfaces de côté de rainure inclinées l'une par rapport à l'autre (92, 94) d'une rainure de stabilisation (58) un angle d'ouverture de rainure (88, 90) qui est situé dans une plage d'environ 30° à environ 60°, en particulier dans une plage d'environ 40° à environ 50°.

6. Instrumentation médicale selon l'une des revendications précédentes, **caractérisée en ce que**
a) la pluralité de rainures de stabilisation (58, 60, 62, 64) présente un fond de rainure arrondi (96, 98)
et/ou
b) une profondeur de rainure de la pluralité de rainures de stabilisation (58) sur la surface de côté latérale (34) est plus grande qu'une profondeur de dent des dents de râpe (50)
et/ou
c) une profondeur de rainure de la pluralité de rainures de stabilisation (60) sur la surface de côté médiane (36) est plus petite qu'une profondeur de dent des dents de râpe (50)
et/ou
d) une profondeur de rainure de la pluralité de rainures de stabilisation (62, 64) sur la surface de côté antérieure (38) et/ou sur la surface de côté postérieure (40) est plus petite qu'une profondeur de dent des dents de râpe (50).

7. Instrumentation médicale selon l'une des revendications précédentes, **caractérisée en ce que** la surface de côté médiane (36) présente un domaine de surface de côté proximal (46) et un domaine de surface de côté distal (48) et **en ce que** le domaine de surface de côté proximal (46) est incurvé de manière concave dans une direction parallèle à l'axe longitudinal (30) en s'écartant de la tige de râpe (14) et est plan ou incurvé de manière convexe dans une direction perpendiculaire à l'axe longitudinal (30) en s'écartant de la tige de râpe (14).

8. Instrumentation médicale selon la revendication 7, **caractérisée en ce que**
a) la pluralité de rainures de stabilisation (60) de la surface de côté médiane (36) sont formées dans le domaine de surface de côté proximal ou sensiblement dans le domaine de surface de côté proximal (46) et/ou
b) la pluralité de rainures de stabilisation (62, 64) de la surface de côté antérieure (38) et/ou de la surface de côté postérieure (40) sont formées du côté distal des domaines de surface de côté proximaux (42, 46) de la surface de côté latérale (34) et/ou de la surface de côté médiane (36).

9. Instrumentation médicale selon l'une des revendications précédentes, **caractérisée en ce que** la surface de côté latérale (34) présente un domaine de surface de côté proximal (42) et un domaine de surface de côté distal (44) et **en ce que** le domaine de surface de côté proximal (42) est incurvé de manière convexe dans une direction parallèle à l'axe longitudinal (30) en s'écartant de la tige de râpe (14) et est plan ou incurvé de manière convexe dans une direction perpendiculaire à l'axe longitudinal (30) en s'écartant de la tige de râpe (14),
où en particulier la pluralité de rainures de stabilisation (58) de la surface de côté latérale (34) est formée dans le domaine de surface de côté proximal ou sensiblement dans le domaine de surface de côté proximal (42).

10. Instrumentation médicale selon l'une des revendications précédentes, **caractérisée en ce que** les dents de râpe (50) présentent une première surface de côté de dent de râpe (100) qui s'étend transversalement ou sensiblement transversalement à l'axe longitudinal (30) et est tournée dans la direction distale, et une seconde surface de côté de dent de râpe (102) qui est inclinée par rapport à l'axe longitudinal (30) et est tournée sensiblement dans la direction proximale.

11. Instrumentation médicale selon la revendication 10, **caractérisée en ce que**
a) les première et seconde surfaces de côté de dent de râpe (100, 102) définissent entre elles un angle de dent de râpe (104) qui est inférieur à 90°, en particulier inférieur à 70°,
et/ou
b) la première surface de côté de dent de râpe (100) est légèrement inclinée dans la direction de l'axe longitudinal (30) pour former une contre-dépouille (106) agissant dans la direction distale.

12. Instrumentation médicale selon l'une des revendications précédentes, **caractérisée en ce que** la tige de râpe (14) comprend une section d'extrémité distale (32) et **en ce que** la section d'extrémité distale (32) de la tige de râpe (14) définit l'axe longitudinal (30).

13. Instrumentation médicale selon l'une des revendications précédentes, **caractérisée en ce que**
a) la surface de côté antérieure (38) et/ou la surface de côté postérieure (40) et/ou la surface de côté latérale (34) et/ou la surface de côté médiane (36) sont formées au moins en partie de manière plane ou de manière sensiblement plane
et/ou
b) un premier élément de couplage (18) pour la liaison amovible avec une poignée de râpe (16) est disposé ou formé sur la tige de râpe (14) dans le domaine de son extrémité proximale.

14. Instrumentation médicale selon l'une des revendications précédentes, **caractérisée en ce que** l'instrument de râpe (10) est formé de manière modulaire et comprend une poignée de râpe (16) qui peut être reliée de manière amovible avec la tige de râpe (14).

15. Instrumentation médicale selon la revendication 14, **caractérisée en ce que** la poignée de râpe (16) comprend un deuxième élément de couplage (24) qui est en prise dans une position de couplage avec le premier élément de couplage (18) et est hors de prise dans une position de nettoyage.
